# EUROPEAN PATENT APPLICATION

(11) **EP 0 831 091 A2**
(43) Date of publication of application: **25.03.1998**
(21) Application number: 97116411.6
(22) Date of filing: 19.09.1997
(51) Int. Cl.: C07D 405/04, C07D 239/54

(54) **Process for the production of 3-aryl-uracils**

(30) Priority: 23.09.1996 CH 2321/96
(71) Applicant: Novartis AG, 4058 Basel (CH)
(72) Inventor: Sting, Andrea Rolf, 5073 Gipf-Oberfrick (CH)
(74) Representative: Roth, Bernhard M.

(57) **Abstract**

Compounds of formula I 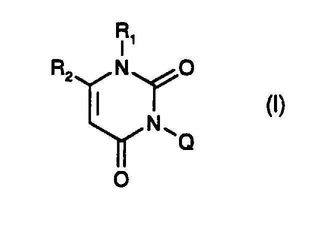 wherein
R₁ signifies methyl or ethyl;
R₂ signifies -CF₃, -CClF₂, -CCl₂F or -C₂F₅;
Q is a group 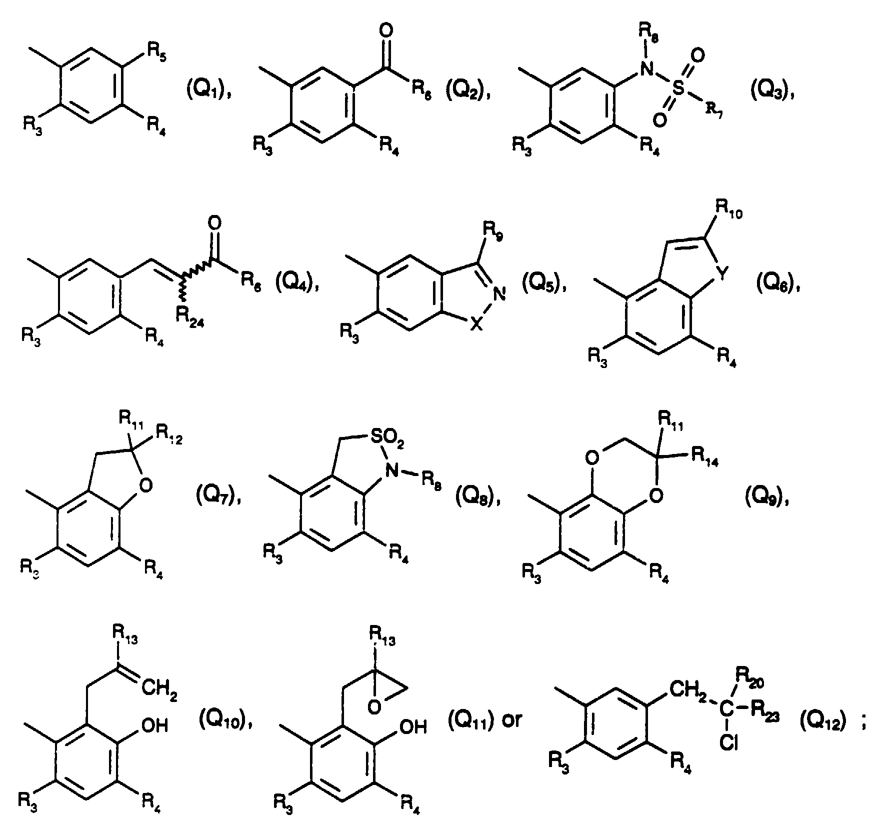 and the remaining substituents have the significance given under formula I, are produced whereby a compound of formula II

   O=C=N-Q (II)

   wherein Q has the significance given under formula I, is reacted at a temperature of -5ºC to +40ºC with an enamine of formula III 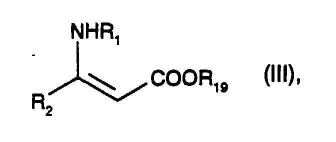 wherein R₁ and R₂ have the significances given under formula I,and R₁₉ signifies C₁-C₆-alkyl, in pure dimethylformamide, dimethyl sulphoxide, acetonitrile, propionitrile, ethyl acetate, tetrahydrofuran, dioxane, N-methylpyrrolidone, methyl-tert.-butylether, dimethylacetamide or toluene or mixtures thereof as solvents, in the presence of 0.2 to 0.4 equivalents of a base selected from potassium tert.butylate, sodium tert.butylate, sodium methylate, sodium ethylate, potassium methylate, potassium ethylate, sodium hydride, potassium hydride, sodium pentylate, potassium pentylate and 1,8-diazabicyclo[5.4.0]undec-7-ene (DBU).

## Description

The present invention relates to a new process for the production of 3-phenyl-uracils, by reacting phenylisocyanates with N-alkyl-enamines.

From WO 95/17931, it is known that compounds of formulae A and B 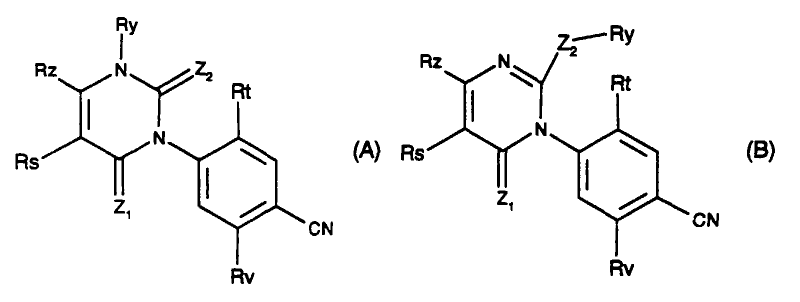 wherein Z₁ and Z₂ denote oxygen or sulphur, whereby at least one of the groupings denotes sulphur; Ry denotes hydrogen or alkyl, alkenyl, alkinyl or alkylcarbonyl, Rs denotes hydrogen, halogen, cyano or optionally substituted alkyl, Rt denotes hydrogen or halogen, Rv denotes halogen, cyano, nitro, amino or an aminoalkylsulphonylalkyl group and Rz denotes alkyl or halogen-alkyl, may be produced in a manner whereby an enamine of formula C 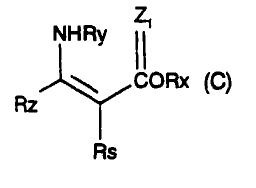 wherein Rx denotes alkyl, is reacted with a cyanoaryliso(thio)cyanate of formula D 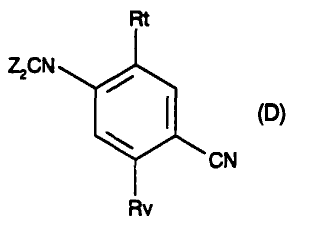 optionally in the presence of a reaction excipient and optionally in the presence of a diluent.

The reactions disclosed specifically in the preparation examples are carried out in a solvent mixture of dimethylformamide/toluene at temperatures of -70ºC or -15ºC, whereby 1 equivalent of sodium hydride is respectively used a base. The yields of only 9 or 25% of theory attained in this reactions are however completely unsatisfactory especially for large scale usage.

It has now surprisingly been found that the yields of such reactions may be increased considerably if the reaction is carried out in a special solvent in the presence of a defined quantity of certain selected bases in a narrowly restricted temperature range especially adapted thereto.

In accordance with the invention, it is therefore proposed that compounds of formula I 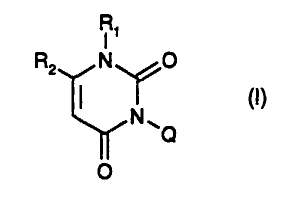 wherein
R₁ signifies methyl or ethyl;
R₂ signifies -CF₃, -CClF₂, -CCl₂F or -C₂F₅;
Q is a group 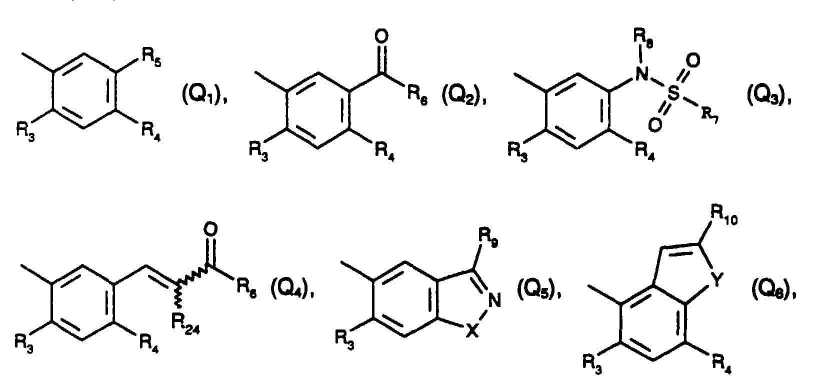 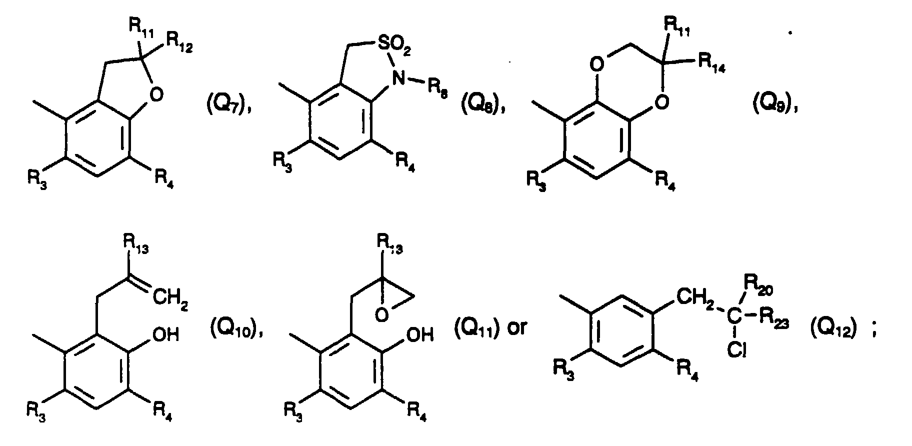
X and Y, independently of one another, are oxygen or sulphur;
R₃ signifies hydrogen, fluorine or chlorine;
R₄ signifies hydrogen, fluorine, chlorine, bromine, cyano, nitro, methyl or difluoromethyl;
R₅ signifies hydrogen, halogen, cyano, nitro, hydroxy, C₁₋₆-alkoxy, C₁₋₆-alkylthio, C₂₋₆-alkenyloxy, C₂₋₆-alkinyloxy, C₁₋₆-halogenalkoxy, C₂₋₆halogen-alkenyloxy, C₂₋₈-alkylcarbonylalkoxy, C₂₋₈-alkoxycarbonylalkoxy, C₁₋₃-alkyl-oxiranylmethoxy, 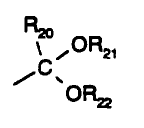 C₄₋₈-alkenyloxycarbonylalkoxy or C₄₋₈-alkinyloxycarbonylalkoxy;
R₂₀ is hydrogen or C₁₋₄-alkyl;
R₂₁ and R₂₂, independently of one another, signify C₁₋₄-alkyl; or R₂₁ and R₂₂ together signify a C₂₋₃-alkylene bridge;
R₂₃ signifies cyano or COR₆;
R₂₄ signifies hydrogen or halogen;
R₆ signifies OH, C₁₋₆-alkoxy, C₂₋₆-alkenyloxy, C₂₋₆-alkinyloxy, C₂₋₈-alkoxyalkoxy, C₃₋₆-cycloalkoxy, C₃₋₆-cycloalkenyloxy, C₃₋₆-cycloalkyl-C₁₋₆-alkoxy, C₁₋₆-halogen-alkoxy, C₂₋₆-halogen-alkenyloxy, C₁₋₆-hydroxycarbonylalkoxy, C₃₋₈-alkoxycarbonylalkoxy, C₃₋₈-alkenyloxycarbonylalkoxy, C₃₋₈-alkinyloxycarbonylalkoxy, N(C₁₋₃-alkyl)₂ or N(C₃₋₄-alkenyl)₂;
R₇ signifies C₁₋₆-alkyl, C₂₋₆-alkenyl, C₂₋₆-alkinyl, C₂₋₈-alkoxyalkyl, C₃₋₆-cycloalkyl, C₁₋₆-halogen-alkyl, C₁₋₆-halogen-alkenyl, C₂₋₆-alkylsulfonyloxyalkyl, C₁₋₁₀-phenylsulfonyloxyalkyl, N(C₁₋₅-alkyl)₂ or diallylamino;
R₈ signifies hydrogen, C₁₋₆-alkyl, C₂₋₆-alkenyl, C₂₋₆-alkinyl, C₂-C₁₀-alkoxyalkyl, C₃₋₆-cycloalkyl, C₃₋₆-cycloalkenyl, C₁₋₆-halogen-alkyl, C₃₋₆-halogen-alkenyl, C₂₋₆-hydroxycarbonylalkyl, C₃₋₈-alkoxycarbonylalkyl, C₃₋₈-alkenyloxycarbonylalkyl or C₃₋₈-alkinyloxycarbonylalkyl;
R₉ signifies hydrogen, OH, CH₂COOR₁₅, CH₂CON(C₁₋₄-alkyl)₂, CH₂CON(C₃₋₄-alkenyl)₂, COOR₁₆, CON(C₁₋₄-alkyl)₂, CON(C₃₋₄-alkenyl)₂, C₁₋₆-alkyl, hydroxy-C₁₋₆-alkyl, C₂₋₁₀-alkoxycarbonyl-alkoxy or C₂₋₈-alkoxyalkyl;
R₁₀ signifies hydrogen, Cyano, C₁₋₆-alkyl, C₂₋₆-alkenyl, C₂₋₆-alkinyl, hydroxy-C₁₋₆-alkyl, C₂₋₈-alkoxyalkyl, C₃₋₆-cycloalkyl, C₁₋₆-halogen-alkyl, COOR₁₇, CON(C₁₋₄-alkyl)₂ or CON(C₃₋₄-alkenyl)₂;
R₁₁ signifies hydrogen, C₁₋₆-alkyl or C₁₋₆-halogen-alkyl;
R₁₂ signifies hydrogen, C₁₋₆-alkyl, C₁₋₆-halogen-alkyl, CH₂OH, C₂₋₆-alkoxyalkyl, C₂₋₆-halogenalkoxyalkyl, COOR₁₈, CON(C₁₋₄-alkyl)₂ or CON(C₃₋₄-alkenyl)₂;
R₁₃ signifies hydrogen, C₃₋₁₆-trialkylsilyloxy, C₁₋₆-alkoxy, chlorine, C₁₋₆-alkyl, C₂₋₆-alkenyl, C₂₋₆-alkinyl, C₃₋₆-cycloalkyl, C₃₋₆-cycloalkenyl, C₁₋₆-halogen-alkyl, C₂₋₆-halogen-alkenyl or C₁₋₆-hydroxycarbonylalkyl;
R₁₄ signifies hydrogen, C₁₋₆-alkyl or C₁₋₆-halogenalkyl;
R₁₅ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₃₋₈-alkoxycarbonylalkyl, C₃₋₈-alkenyloxycarbonylalkyl or C₃₋₈-alkinyloxycarbonylalkyl;
R₁₆ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₃₋₈-alkoxycarbonylalkyl, C₃₋₈-alkenyloxycarbonylalkyl or C₃₋₈-alkinyloxycarbonylalkyl;
R₁₇ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₂₋₈-alkoxycarbonylalkyl, C₃₋₈-alkenyloxycarbonylalkyl or C₃₋₈-alkinyloxycarbonylalkyl; and
R₁₈ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₃₋₈-alkoxycarbonylalkyl, C₃₋₈-alkenyloxycarbonylalkyl or C₃₋₈-alkinyloxycarbonylalkyl, are produced in a manner whereby a compound of formula II

   O=C=N-Q (II)

   wherein Q has the significance given under formula I, is reacted at a temperature of -5ºC to +40ºC with an enamine of formula III 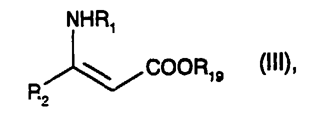 wherein R₁ and R₂ have the significances given under formula I,and R₁₉ signifies C₁-C₆-alkyl, in pure dimethylformamide, dimethyl sulphoxide, acetonitrile, propionitrile, ethyl acetate, tetrahydrofuran, dioxane, N-methylpyrrolidone, methyl-tert.-butylether, dimethylacetamide or toluene or mixtures thereof as solvents, in the presence of 0.2 to 0.4 equivalents of a base selected from potassium tert.butylate, sodium tert.butylate, sodium methylate, sodium ethylate, potassium methylate, potassium ethylate, sodium hydride, potassium hydride, sodium pentylate, potassium pentylate and 1,8-diazabicyclo[5.4.0]undec-7-ene (DBU), preferably potassium tert.butylate and potassium tert.pentylate.

The alkyl groups present in the definitions of substituents may be straight-chain or branched, and denote for example methyl, ethyl, propyl, butyl, pentyl, hexyl, heptyl, octyl, nonyl or decyl, as well as the branched isomers thereof. Alkoxy, alkylthio, halogen-alkyl, alkylamino, alkenyl and alkinyl groups are derived from the said alkyl groups. The alkenyl and alkinyl groups may be unsaturated once or many times. For definitions such as C₃₋₈-alkinylcarbonylalkyl, the carbonyl atom is not counted as a carbon atom.

Appropriate cycloalkyl substituents contain 3 to 6 carbon atoms and are e.g. cyclopropyl, cyclobutyl, cyclopentyl or cyclohexyl. Corresponding cycloalkenyl substituents may be unsaturated once or also many times, as for example cyclopentadienyl or cyclohexadienyl.

Halogen normally signifies fluorine, chlorine, bromine or iodine. The same also applies to halogen in conjunction with other significances, e.g. halogen-alkyl.

The preferred base in the process according to the invention is potassium tert.butylate. The base is most preferably used in an amount of 0.25 to 0.35, especially 0.3 equivalents in relation to the employed enamine of formula III. An especially preferred temperature range is 0ºC to +20ºC, especially 0ºC to +5ºC.

Addition of the reagents may be effected in various ways. For example, the base may be presented in pure dimethylformamide, dimethyl sulphoxide, acetonitrile, propionitrile, ethyl acetate, tetrahydrofuran, dioxane, N-methylpyrrolidone, methyl-tert.-butylether, dimethylacetamide or toluene or mixtures thereof, the compound of formula III added and subsequently the compound of formula II introduced, or the compound of formula III and II may be presented as a mixture and subsequently the base in pure dimethylformamide, dimethyl sulphoxide, acetonitrile, propionitrile, ethyl acetate, tetrahydrofuran, dioxane, N-methylpyrrolidone, methyl-tert.-butylether, dimethylacetamide or toluene or mixtures thereof is added, or the base is presented in pure dimethylformamide, dimethyl sulphoxide, acetonitrile, propionitrile, ethyl acetate, tetrahydrofuran, dioxane, N-methylpyrrolidone, methyl-tert.-butylether, dimethylacetamide or toluene or mixtures thereof and subsequently a mixture of compound of formula III and II added. In a preferred variant of the process according to the invention, the base is presented in pure dimethylformamide, dimethyl sulphoxide, acetonitrile, propionitrile, ethyl acetate, tetrahydrofuran, dioxane, N-methylpyrrolidone, methyl-tert.-butylether, dimethylacetamide or toluene or mixtures thereof and then the compound of formula III is added, and afterwards the compound of formula II.

In order to attain the desired high selectivity of the reaction, the enamine of formula III is advantageously used in excess, and preferably 2 equivalents of enamine of formula III are brought to react with 1 equivalent of the isocyanate of formula II.

In the process according to the invention, preferably those compounds of formula I are produced wherein R₁ signifies methyl or ethyl; R₂ signifies -CF₃, -CClF₂, -CCl₂F or -C₂F₅;
Q is a group 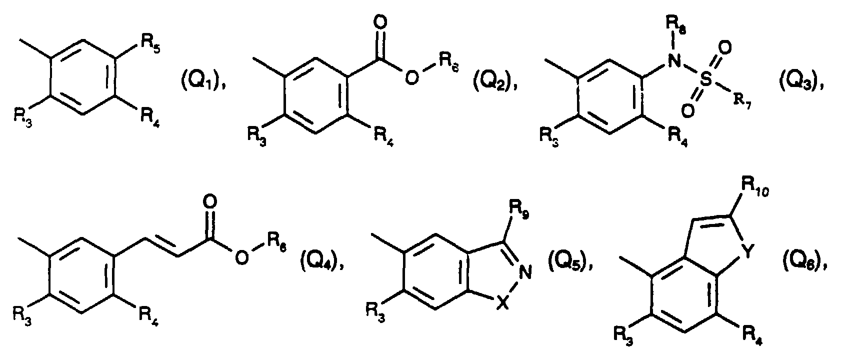 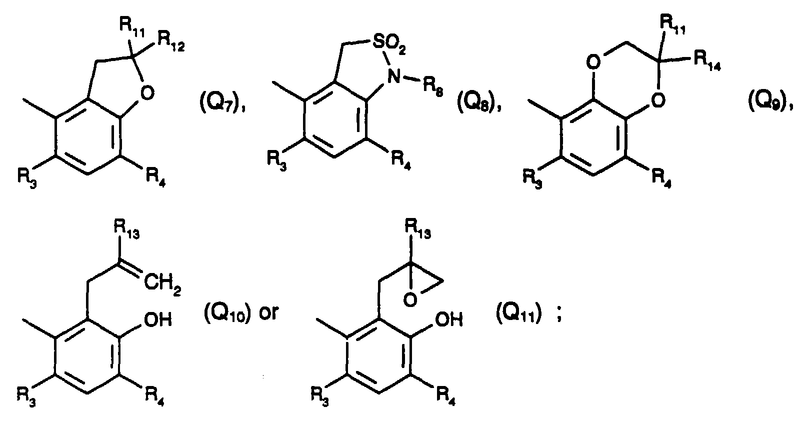
X and Y, independently of one another, are oxygen or sulphur;
R₃ signifies hydrogen, fluorine or chlorine;
R₄ signifies hydrogen, fluorine, chlorine, bromine, cyano, nitro, methyl or difluoromethyl;
R₅ signifies hydrogen, halogen, cyano, nitro, hydroxy, C₁₋₆-alkoxy, C₂₋₆-alkenyloxy, -C₂₋₆-alkinyloxy, C₁₋₆-halogen-alkoxy, C₂₋₆-halogen-alkenyloxy, C₃₋₈-alkoxycarbonylalkoxy or C₁₋₆-alkylthio;
R₆ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₁₋₆-hydroxycarbonylalkyl, C₃₋₈-alkoxycarbonylalkyl, C₃₋₈-alkenyloxycarbonylalkyl, C₃₋₈-alkinylcarbonylalkyl, C₂₋₆-dialkyleneamino or C₃₋₈-dialkyleneaminooxyalkyl;
R₇ signifies C₁₋₆-alkyl, C₂₋₆-alkenyl, C₂₋₆-alkinyl, C₂₋₈-alkoxyalkyl, C₃₋₆-cycloalkyl, C₁₋₆-halogen-alkyl, C₁₋₆-halogen-alkenyl, C₂₋₆-alkylsulfonyloxyalkyl, C₁₋₁₀-phenylsulfonyloxyalkyl, C₂₋₁₀-dialkylamino or diallylamino;
R₈ signifies hydrogen, C₁₋₆-alkyl, C₂₋₆-alkenyl, C₂₋₆-alkinyl, C₃₋₆-cycloalkyl, C₃₋₆-cycloalkenyl, C₁₋₆-halogen-alkyl, -C₁₋₆-halogen-alkenyl, C₂₋₆-hydroxycarbonylalkyl, C₃₋₈-alkoxycarbonylalkyl, C₂₋₈-alkenyloxycarbonylalkyl or C₃₋₈-alkinylcarbonylalkyl;
R₉ signifies hydrogen, CH₂COOR₁₅, COOR₁₆, C₁₋₆-alkyl or C₂₋₈-alkoxyalkyl;
R₁₀ signifies hydrogen, C₁₋₆-alkyl, C₂₋₆-alkenyl, C₂₋₆-alkinyl, C₂₋₈-alkoxyalkyl, C₃₋₆-cycloalkyl, C₁₋₆-halogen-alkyl or COOR₁₇;
R₁₁ signifies hydrogen, C₁₋₆-alkyl or C₁₋₆-halogen-alkyl;
R₁₂ signifies hydrogen, C₁₋₆-alkyl, C₁₋₆-halogen-alkyl, CH₂OH, C₂₋₆-alkoxyalkyl, C₂₋₆-halogen-alkoxyalkyl or COOR₁₈;
R₁₃ signifies hydrogen, hydroxy, C₃₋₁₆-trialkylsilyloxy, C₁₋₆-alkoxy, chlorine, C₁₋₆-alkyl, C₂₋₆-alkenyl, C₂₋₆-alkinyl, C₃₋₆-cycloalkyl, C₃₋₆-cycloalkenyl, C₁₋₆-halogen-alkyl, C₂₋₆-halo-alkenyl or C₁₋₆-hydroxycarbonylalkyl;
R₁₄ signifies hydrogen, C₁₋₆-alkyl or C₁₋₆-halogen-alkyl;
R₁₅ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₃₋₈-alkoxycarbonylalkyl, C₃₋₈-alkenyloxycarbonylalkyl, C₃₋₈-alkinylcarbonylalkyl, C₂₋₆-dialkylamino, C₂₋₈-dialkyleneamino or C₃₋₈-dialkyleneaminooxyalkyl;
R₁₆ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₃₋₈-alkoxycarbonylalkyl, C₃₋₈-alkenyloxycarbonylalkyl, C₃₋₈-alkinylcarbonylalkyl, C₂₋₆-dialkylamino, C₂₋₈-dialkyleneamino or C₃₋₈-dialkyleneaminooxyalkyl;
R₁₇ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₃₋₈-alkoxycarbonylalkyl, C₃₋₈-alkenyloxycarbonylalkyl, C₃₋₈-alkinylcarbonylalkyl, C₂₋₆-dialkylamino, C₂₋₈-dialkyleneamino or C₃₋₈-dialkyleneaminooxyalkyl; and
R₁₈ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₃₋₈-alkoxycarbonylalkyl, C₃₋₈-alkenyloxycarbonylalkyl, C₃₋₈-alkinylcarbonylalkyl, C₂₋₆-dialkylamino, C₂₋₈-dialkyleneamino or C₃₋₈-dialkyleneaminooxyalkyl,
   and it is characterised in that a compound of formula II

   O=C=N-Q (II),

   wherein Q has the significance given under formula I, is reacted at a temperature of -5°C to +40°C with an enamine of formula III 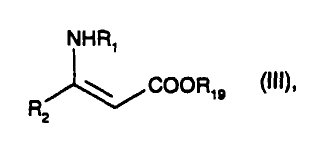 wherein R₁ and R₂ have the significance given under formula I and R₁₉ signifies C₁-C₆-alkyl, in pure dimethylformamide or dimethyl sulphoxide as the solvent and in the presence of 0.2 to 0.4 equivalents of a base selected from potassium tert.butylate, sodium methylate and sodium hydride.

In the process according to the invention, advantageously those compounds of formula I are produced wherein Q is Q₁, Q₂, Q₃ or Q₄. The process according to the inventon is especially suitable for the production of the following compounds:
3-(2,5-difluoro-phenyl)-1-methyl-6-trifluoromethyl-1H-pyrimidine-2,4-dione,
3-(2,4-difluoro-phenyl)-1-methyl-6-trifluoromethyl-1H-pyrimidine-2,4-dione,
3-(4-chloro-2-fluoro-phenyl)-1-methyl-6-trifluoromethyl-1H-pyrimidine-2,4-dione,
3-(5-bromo-4-chloro-2-fluoro-phenyl)-1-methyl-6-trifluoromethyl-1H-pyrimidine-2,4-dione,
3-(4-chloro-2-fluoro-5-nitro-phenyl)-1-methyl-6-trifluoromethyl-1H-pyrimidine-2,4-dione,
3-(4-chloro-5-cyano-2-fluoro-phenyl)-1-methyl-6-trifluoromethyl-1H-pyrimidine-2,4-dione,
3-(5-methallyloxy-4-chloro-2-fluoro-phenyl)-1-methyl-6-trifluoromethyl-1H-pyrimidine-2,4-dione,
3-[4-chloro-2-fluoro-5-(2-methyl-oxiranylmethoxy)-phenyl]-1-methyl-6-trifluoromethyl-1H-pyrimidine-2,4-dione,
2-chloro-5-(3-methyl-2,6-dioxo-4-trifluoromethyl-3,6-dihydro-2H-pyrimidin-1-yl)-benzoic acid isopropyl ester,
2-chloro-4-fluoro-5-(3-methyl-2,6-dioxo-4-trifluoromethyl-3,6-dihydro-2H-pyrimidin-1-yl)-benzoic acid isopropyl ester,
2-chloro-5-(3-methyl-2,6-dioxo-4-trifluoromethyl-3,6-dihydro-2H-pyrimidin-1-yl)-benzoic acid 1-cyclopropyl-ethyl ester,
2-chloro-5-(3-methyl-2,6-dioxo-4-trifluoromethyl-3,6-dihydro-2H-pyrimidin-1-yl)-benzoic acid 1-methoxycarbonyl-ethyl ester,
2-chloro-5-(3-methyl-2,6-dioxo-4-trifluoromethyl-3,6-dihydro-2H-pyrimidin-1-yl)-benzoic acid 2-ethoxycarbonyl-1-methyl-ethyl ester,
2-chloro-5-(3-methyl-2,6-dioxo-4-trifluoromethyl-3,6-dihydro-2H-pyrimidin-1-yl)-benzoic acid 1-carboxy-1-methyl-ethyl ester,
2-chloro-5-(3-methyl-2,6-dioxo-4-trifluoromethyl-3,6-dihydro-2H-pyrimidin-1-yl)-benzoic acid 1-methyl-1-(1-methyl-allyloxycarbonyl)-ethyl ester,
2-chloro-5-(3-methyl-2,6-dioxo-4-trifluoromethyl-3,6-dihydro-2H-pyrimidin-1-yl)-benzoic acid 1-methoxycarbonyl-1-methyl-ethyl ester,
N-[2-chloro-4-fluoro-5-(3-methyl-2,6-dioxo-4-trifluoromethyl-3,6-dihydro-2H-pyrimidin-1-yl)-phenyl]-N-methyl-methanesulfonamide,
N-allyl-N-[2-chloro-4-fluoro-5-(3-methyl-2,6-dioxo-4-trifluoromethyl-3,6-dihydro-2H-pyrimidin-1-yl)-phenyl]-methanesulfonamide,
Ethanesulfonic acid [2-chloro-4-fluoro-5-(3-methyl-2,6-dioxo-4-trifluoromethyl-3,6-dihydro-2H-pyrimidin-1-yl)-phenyl]-ethyl-amide,
C-chloro-N-[2-chloro-4-fluoro-5-(3-methyl-2,6-dioxo-4-trifluoromethyl-3,6-dihydro-2H-pyrimidin-1-yl)-phenyl]-N-prop-2-ynyl-methanesulfonamide,
3-[2-chloro-4-fluoro-5-(3-methyl-2,6-dioxo-4-trifluoromethyl-3,6-dihydro-2H-pyrimidin-1-yl)-phenyl]-acrylic acid isopropyl ester,
3-[2-chloro-4-fluoro-5-(3-methyl-2,6-dioxo-4-trifluoromethyl-3,6-dihydro-2H-pyrimidin-1-yl)-phenyl]-acrylic acid ethyl ester,
3-[2-chloro-4-fluoro-5-(3-methyl-2,6-dioxo-4-trfluoromethyl-3,6-dihydro-2H-pyrimidin-1-yl)-phenyl]-acrylic acid allyl ester and
2-chloro-5-(3,6-dihydro-2,6-dioxo-3-methyl-4-trifluoromethyl-1-(2H)pyrimidinyl)-benzoic acid 1-allyloxycarbonyl-1-methyl-ethylester.

Compounds of formula I are known from the following publications:
US-A-5,183,492, EP-A-0 255 047, EP-A-0 195 346, WO 88/10254, EP-A-0 563 384, US-A-5,523,278, WO 93/06090, EP-A-0 561 319, US-A-5,169,431, EP-A-0 617 033, WO 95/33746, US-A-5,441,925, EP-A-0 705 829 and WO 95/17391.

Compounds of formula II are known for example from US-A-5,183,492 and EP-A-0 195 346.

The enamines of formula III may be produced for example whereby a compound of formula IV 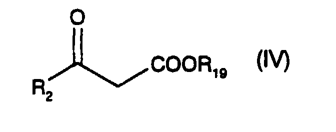 is reacted with a compound of formula V

H-N-R₁ (V)

Reactions of this type are described for example in JP 05140060-A.

### Preparation examples:

### Example H1: Preparation of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester

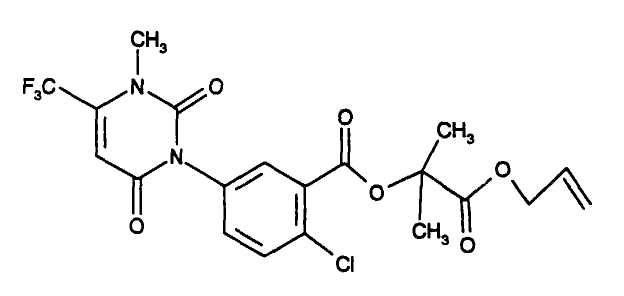

0.347 g of potassium tert.butylate (content: 97%, 0.003 mols) are suspended in 30 g of anhydrous dimethylformamide (water content < 0.01%), and heated to a temperature of 40°C. Subsequently, 1.97 g of 3-methylamino-4,4,4-trifluorobut-2-enoic acid ethyl ester (content: 99.5%, 0.01 mols) are added at a temperature of 40 to 45°C, and this temperature is maintained for a further 30 minutes, whereby a red-brown solution is produced. After cooling the reaction mixture to a temperature of 0°C, 3.51 g of 5-isocyanato-2-chloro-benzoic acid 1-allyloxycarbonyl-1-methyl-ethyl-ester (content: 97%, 0.0105 mols) are dispensed in over the course of 30 minutes, whereby the temperature is maintained at between 0 and 5°C. After removing the cooling bath, the resultant dark brown solution is stirred for a further 30 minutes, and the pH value of the now dark red solution is adjusted to pH 7 with 2.7 g of 1 M HCl [the content of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester in this solution is 11%, which corresponds to a yield of 89%]. The solvent is subsequently distilled off in a rotary evaporator, and the residue (5.6 g) is recrystallised in 12 g of isopropyl alcohol [the inorganic salts are removed by hot filtration]. After drying in a vacuum drying chamber (60°C for 12 hours), 4.10 g (86.3% of theory) of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester are obtained in a content of 99%.

### Example H2: Preparation of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester

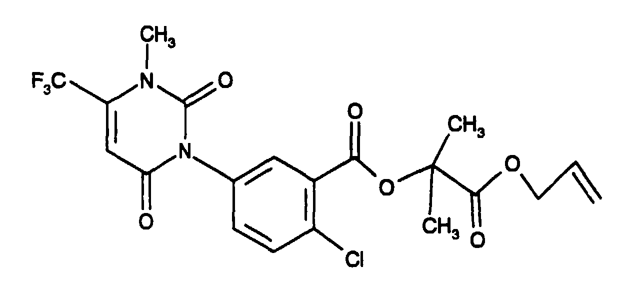

0.347 g of potassium tert.butylate (content: 97%, 0.003 mols) are suspended in 20 g of anhydrous dimethylformamide (water content < 0.01%), and heated to a temperature of 40°C. Subsequently, 1.97 g of 3-methylamino-4,4,4-trifluorobut-2-enoic acid ethyl ester (content: 99.5%, 0.01 mols) are added at a temperature of 40 to 45°C, and this temperature is maintained for a further 30 minutes, whereby a red-brown solution is produced. After cooling the reaction mixture to a temperature of 0°C, 3.51 g of 5-isocyanato-2-chloro-benzoic acid 1-allyloxycarbonyl-1-methyl-ethyl-ester (content: 97%, 0.0105 mols) are dispensed in over the course of 30 minutes, whereby the temperature is maintained at between 0 and 5°C. After removing the cooling bath, the resultant dark brown solution is stirred for a further 30 minutes, and the pH value of the now dark red solution is adjusted to pH 7 with 2.7 g of 1 M HCl [the content of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester in this solution is 13.1%, which corresponds to a yield of 79%]. The solvent is subsequently distilled off in a rotary evaporator, and the residue (5.4 g) is recrystallised in 12 g of isopropyl alcohol [the inorganic salts are removed by hot filtration]. After drying in a vacuum drying chamber (60°C for 12 hours), 3.68 g (77.5% of theory) of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester are obtained in a content of 98.6%.

### Example H3: Preparation of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester

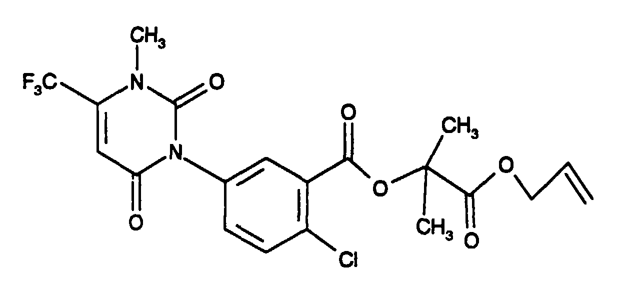

A suspension of 20 g of dry dimethylformamide (water content < 0.01%) and 0.347 g of potassium tert.butylate (content: 97%, 0.003 mols) is added at a temperature of 0 to 5°C to a mixture of 1.97 g of 3-methylamino-4,4,4-trifluorobut-2-enoic acid ethyl-ester (content: 99.5%, 0.01 mols) and 3.51 g of 5-isocyanato-2-chloro-benzoic acid 1-allyloxycarbonyl-1-methyl-ethylester (content: 97%, 0.0105 mols) in such a manner that the temperature of the reaction mixture does not exceed 5°C. The pH value of the resultant dark red solution is adjusted to pH 7 with 2.6 g of 1 M HCl [the content of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester in this solution is 11.2%, which corresponds to a yield of 61.4%]. The solvent is subsequently distilled off in a rotary evaporator, and the residue (5.5 g) is recrystallised in 10 g of isopropyl alcohol [the inorganic salts are removed by hot filtration]. After drying in a vacuum drying chamber (60°C for 12 hours), 2.75 g (58.1% of theory) of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester are obtained in a content of 98.2%.

### Example H4: Preparation of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester

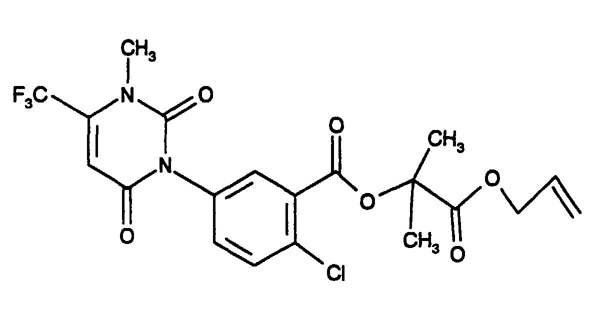

A mixture of 1.97 g of 3-methylamino-4,4,4-trifluorobut-2-enoic acid ethyl-ester (content: 99.5%, 0.01 mols) and 3.51 g of 5-isocyanato-2-chloro-benzoic acid 1-allyloxycarbonyl-1-methyl-ethyl-ester (content: 97%, 0.0105 mols) is added to a suspension of 20 g of dry dimethylformamide (water content < 0.01%) and 0.347 g of potassium tert.butylate (content: 97%, 0.003 mols) in such a manner that the temperature does not exceed 30°C. The pH value of the resultant dark red solution is adjusted to pH 7 with 2.8 g of 1 M HCl [the content of 2-chloro-5-(3,5-dihydro-2,6-dioxo-3-methyl-4-trifluoromethyl-1-(2H)pyrimidinyl)-benzoic acid 1-allyloxycarbonyl-1-methyl-ethyl-ester in this solution is 10.8%, which corresponds to a yield of 59.2%]. The solvent is subsequently distilled off in a rotary evaporator, and the residue (5.5 g) is recrystallised in 10 g of isopropyl alcohol [the inorganic salts are removed by hot filtration]. After drying in a vacuum drying chamber (60°C for 12 hours), 2.75 g (58.1% of theory) of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester are obtained in a content of 98.6%.

### Example H5: Preparation of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester

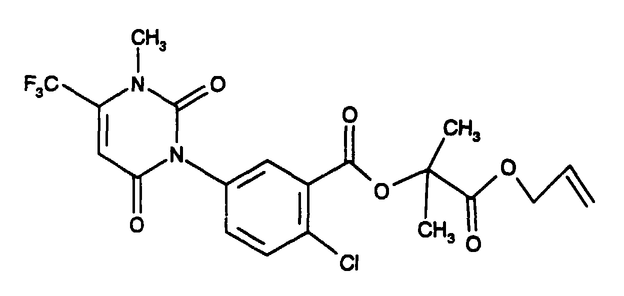

1 g (0.0086 mols) of potassium tert.butylate is added whilst stirring, at room temperature, to a solution of 90 g of acetonitrile (water content < 0.02%) and 17.7 g (0.088 mols) of 3-methylamino-4,4,4-trifluorobut-2-enoic acid ethyl-ester. A clear, orange-red solution is produced, which is heated to 30ºC. 14:3 g (0.044 mols) of 5-isocyanato-2-chloro-benzoic acid 1-allyloxycarbonyl-1-methyl-ethyl-ester in 28 g of acetonitrile are dispensed into this solution over 15 minutes, whereby the colour changes to dark green and afterwards to red. After this dispensing has taken place, the reaction mixture is neutralised by adding 0.6 g (0.0091 mols) of acetic acid [the content of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester in this solution is 11.1%, which corresponds to a yield of 89%]. Subsequently, the solvent is distilled off at 50-55ºC and at 200-250 mbar pressure. Excess 3-methylamino-4,4,4-trifluorobut-2-enoic acid ethyl-ester is separated at 100-130ºC and at 5-10 mbar pressure, and the resulting oily residue (22.2 g) is taken up in 35 g of isopropanol. At 40ºC, the salts are dissolved by adding 10 g of water, and the lower aqueous phase (10.5 g) is separated. The product is crystallised after adding a further 10 g of water at 30ºC, and afterwards cooled to 5ºC. After filtration and drying the filter cake in a vacuum drying chamber (60ºC for 12 hours), 18 g (85% of theory) of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester are obtained in a content of 98.5%.

### Example H6: Preparation of 2-chloro-5-(3,6-dihydro-2,6-dioxo-3-methyl-4-trifluoromethyl-1-(2H)pyrimidinyl)-benzoic acid 1-allyloxycarbonyl-1-methyl-ethyl-ester

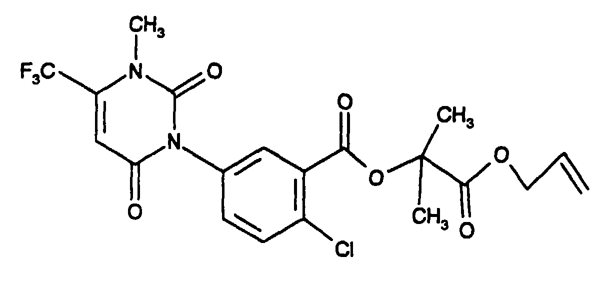

2.9 g of potassium tert.butylate (content 97%, 0.025 mols) are suspended in 169 g of dry acetonitrile (water content < 0.01%) and cooled to 0-5ºC. Subsequently, at 0-5ºC, 36.2 g of 3-methylamino-4,4,4-trifluorobut-2-enoic acid isopropylester (content 98.3%, 0.17 mols) are added and maintained at this temperature for 30 minutes, whereby a red-brown solution is produced. Then, over the course of 15 minutes, at 0-5ºC, 27.3 g of 5-isocyanato-2-chlorobenzoic acid 1-allyloxycarbonyl-1-methyl-ethyl-ester (content 99%, 0.084 mols) are dispensed in, whereby a dark brown solution is obtained. The latter is adjusted to pH 7 with 3.0 g of 1 N hydrochloric acid [content of desired product in the solution is 15.5%, which corresponds to a yield of 91.2%]. The solvent is subsequently distilled off in a vacuum, and the residue (68 g) is recrystallised in 200 g of isopropyl alcohol, whereby the inorganic salts are removed by hot filtration. After drying in a vacuum drying chamber at 60°C for 12 hours, 32.8 g (82% of theory) of the desired product are isolated in a content of 98.7%.

The compounds of formula I listed in the following tables may be produced in analogous manner:

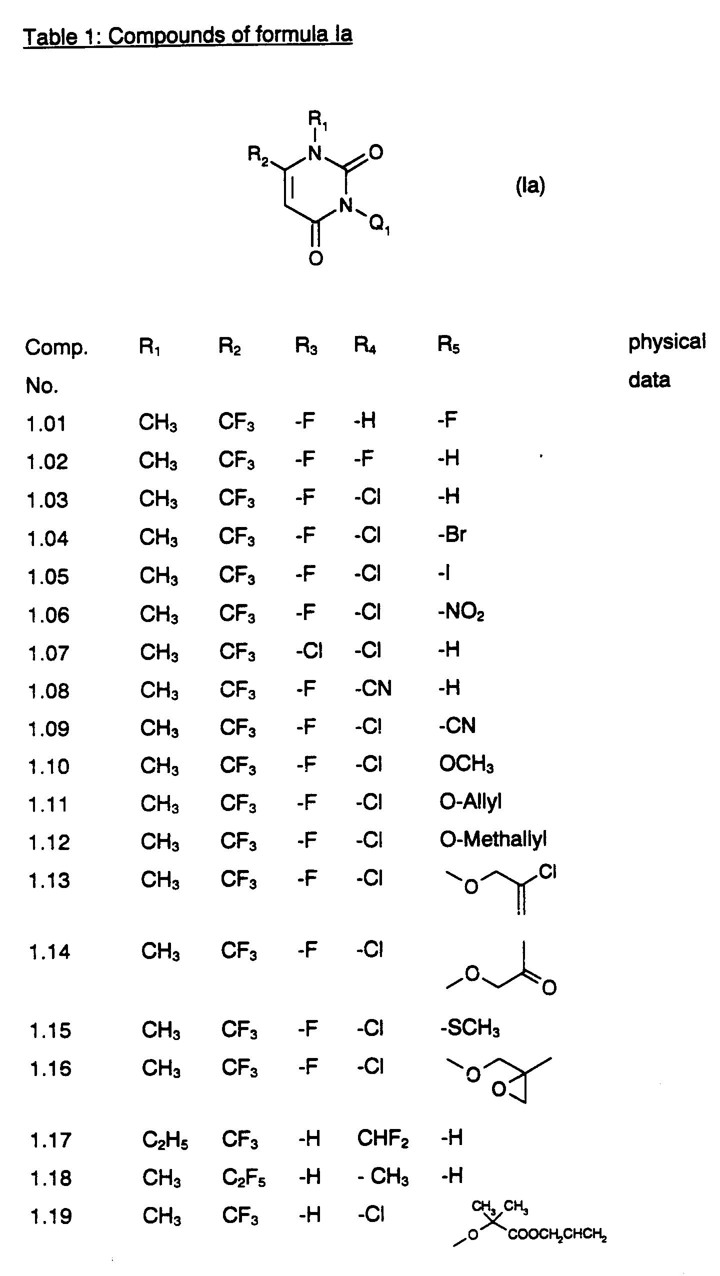

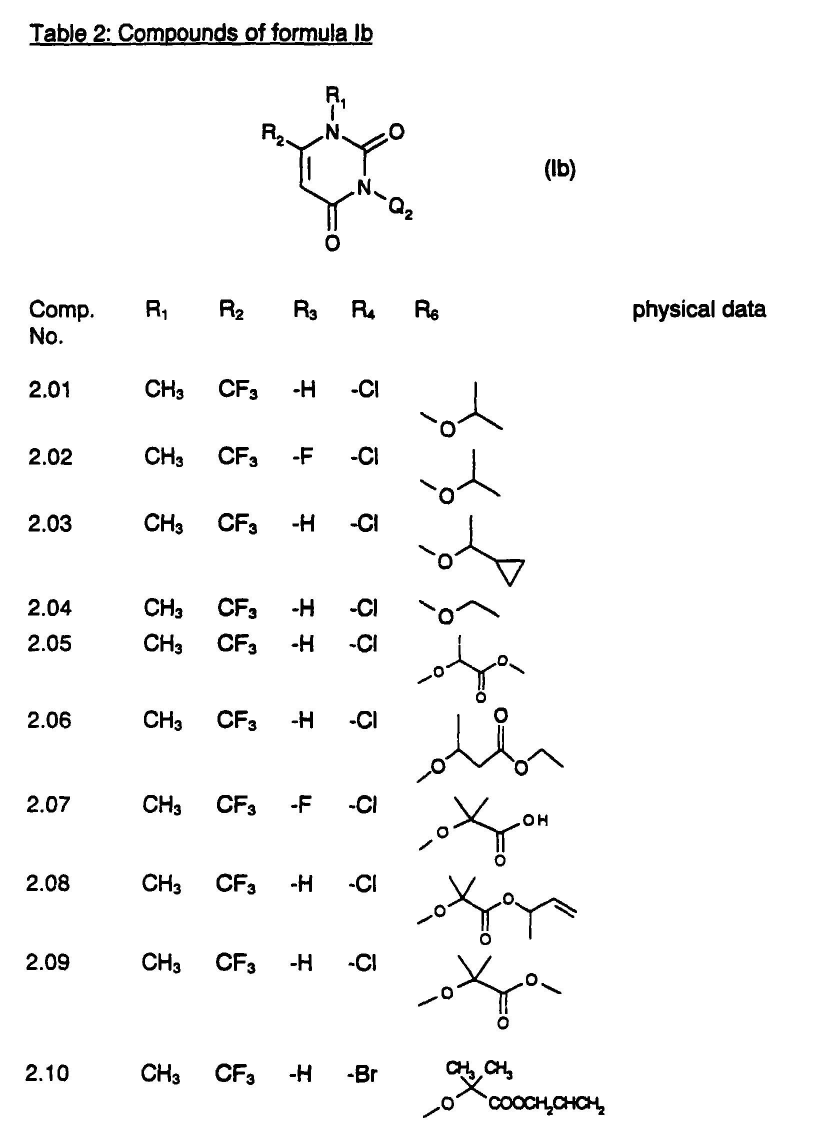

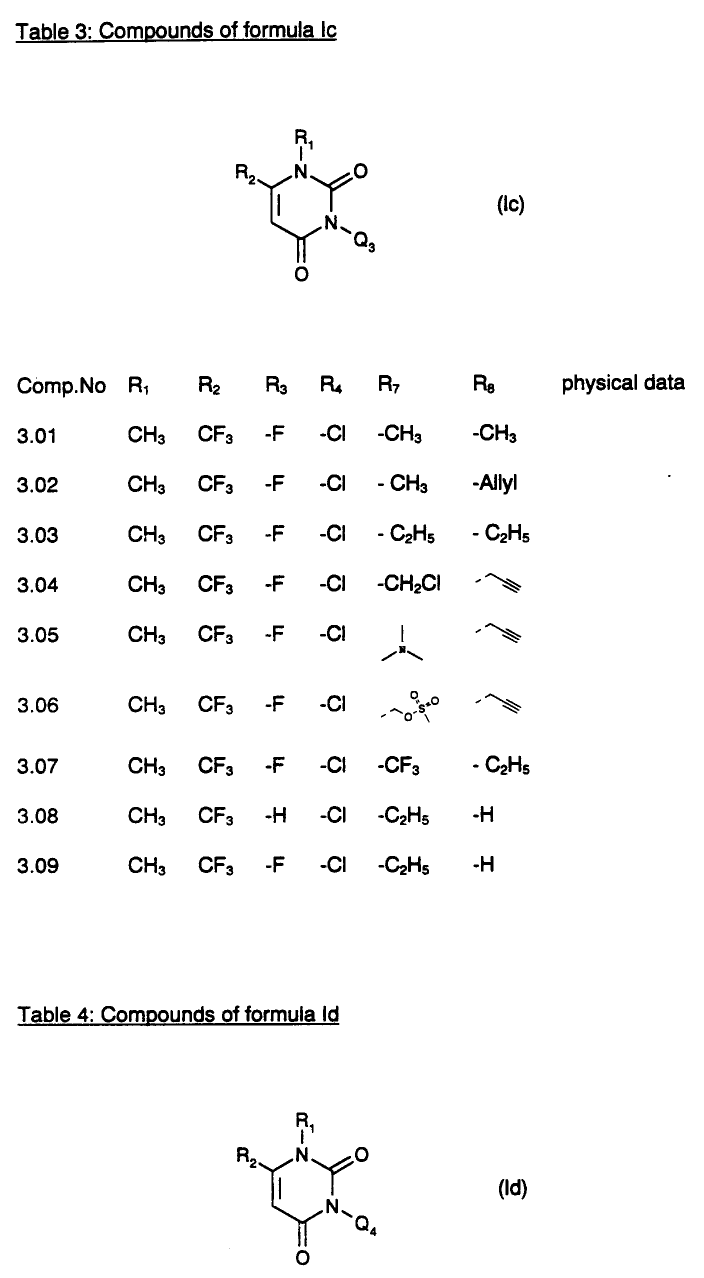

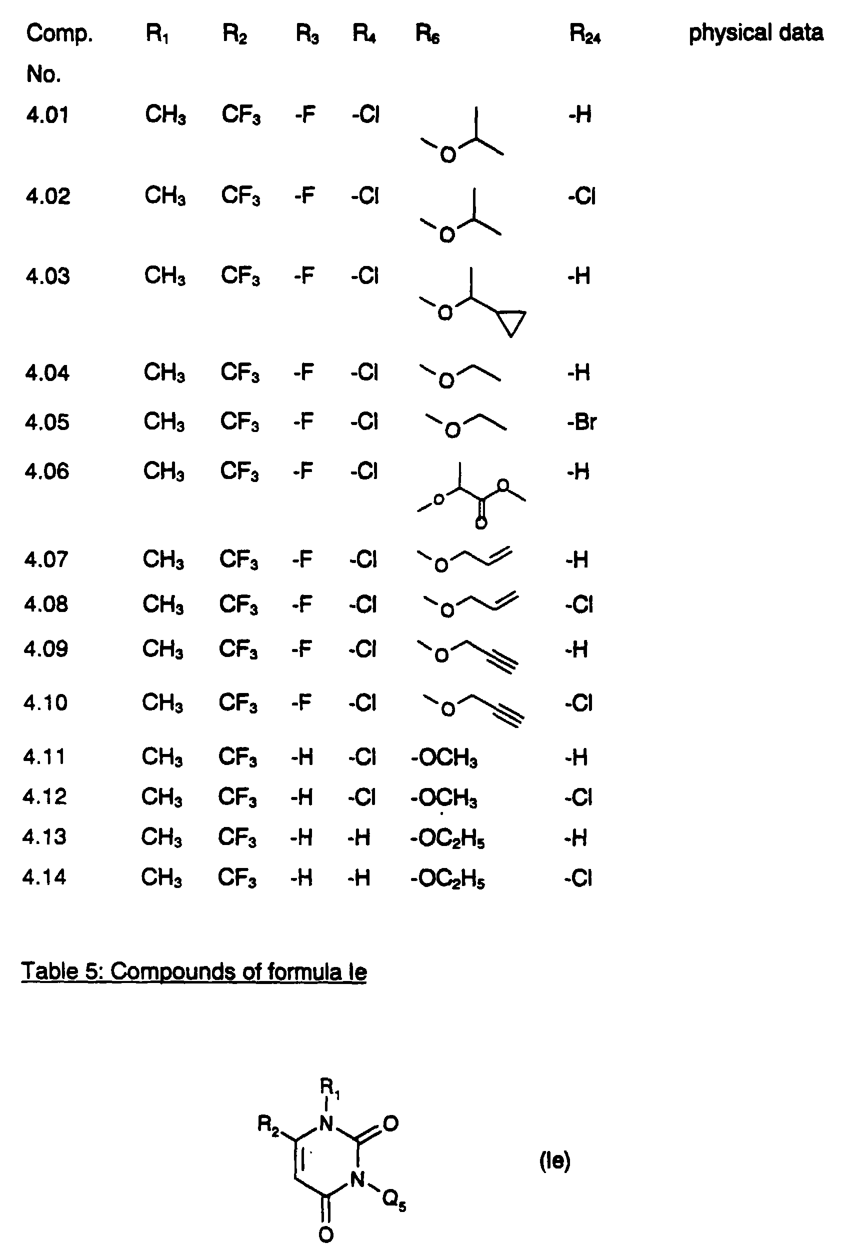

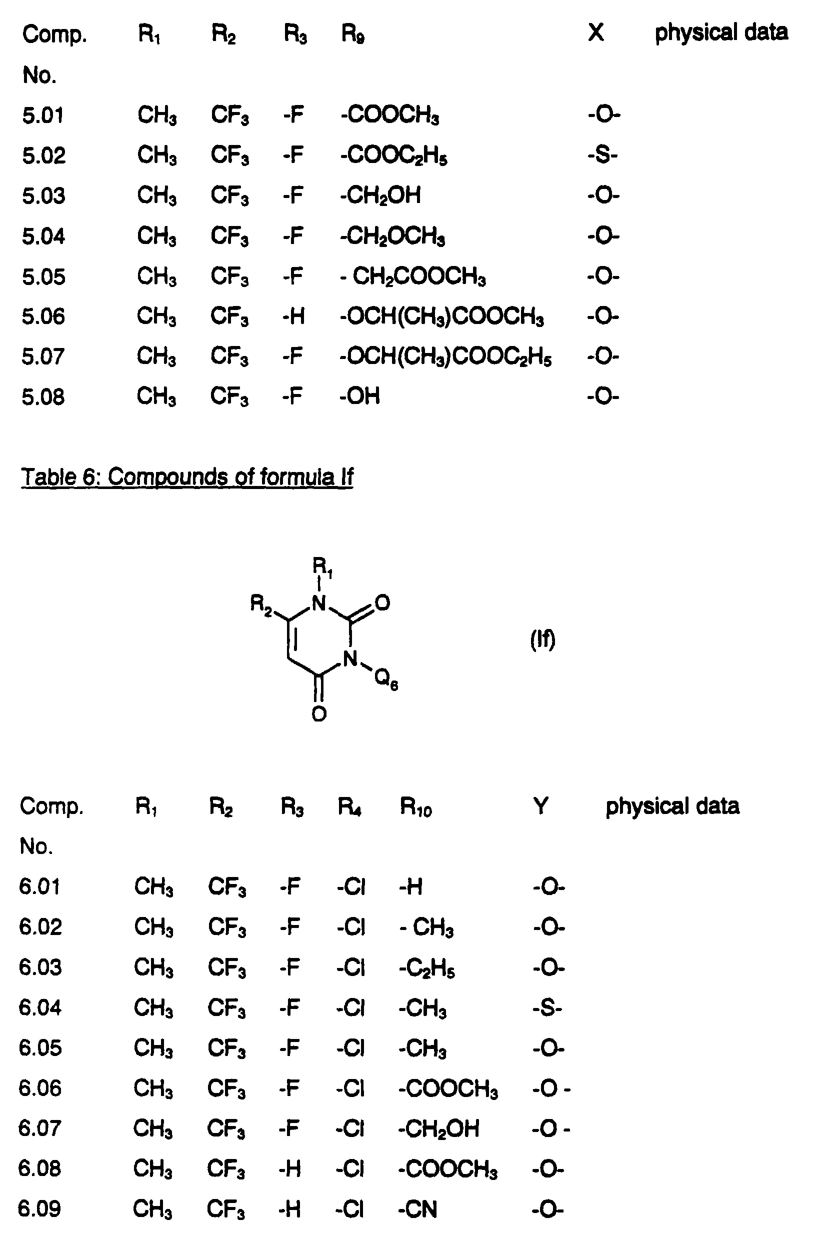

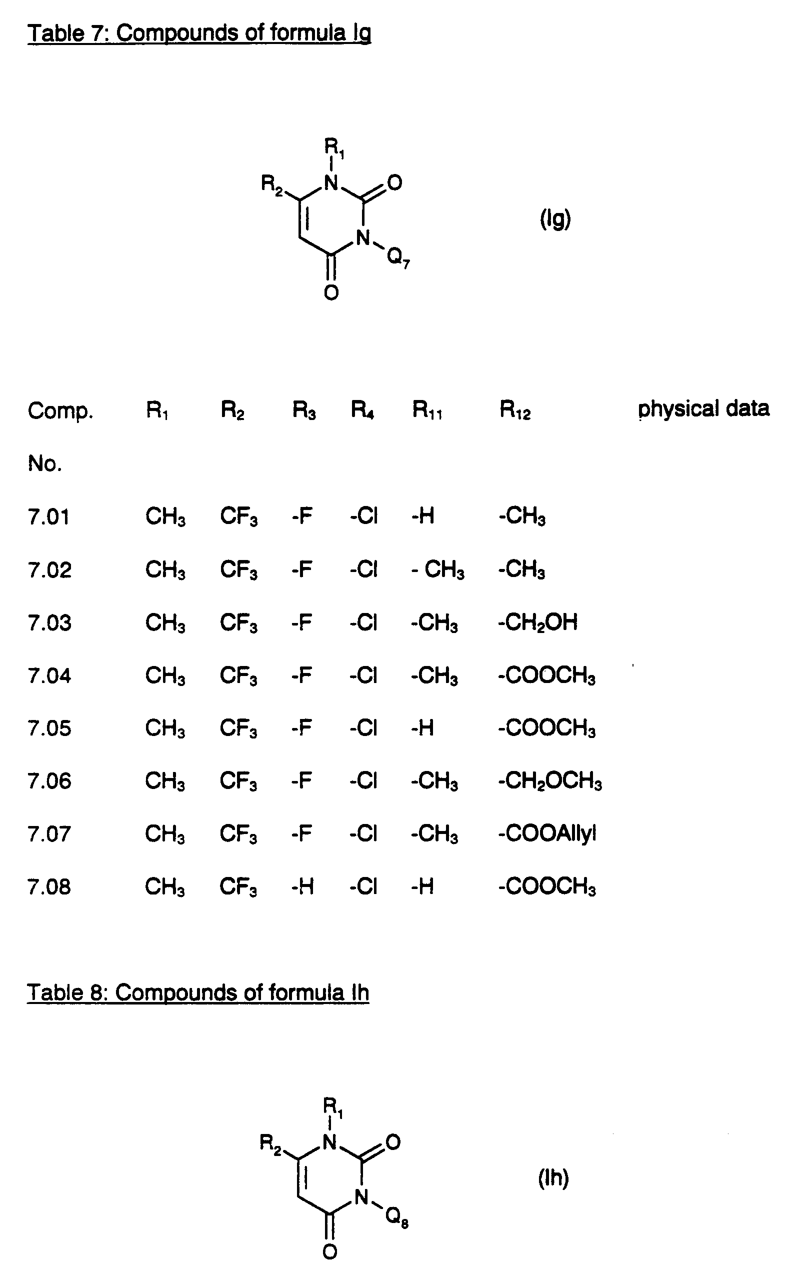

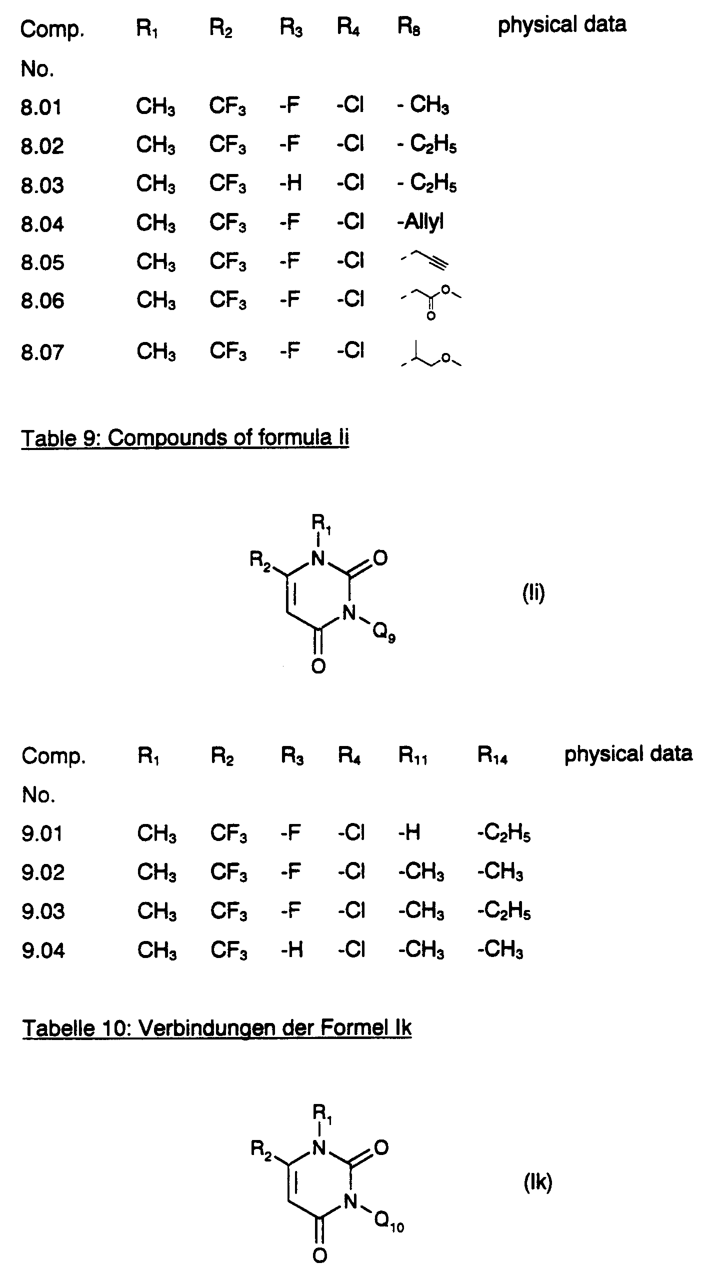

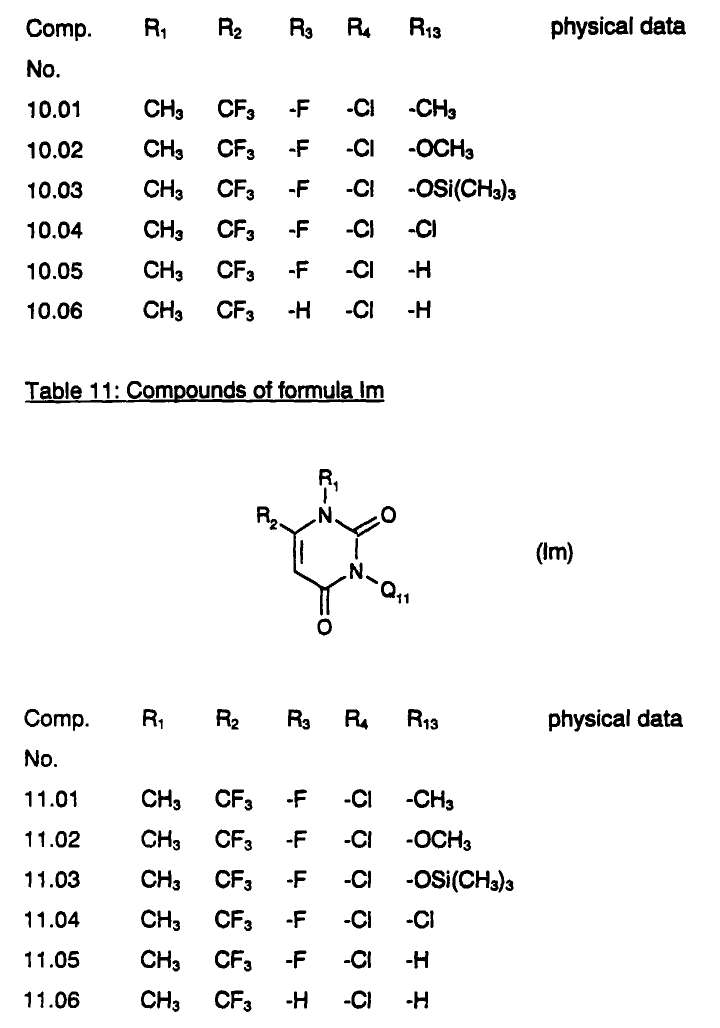

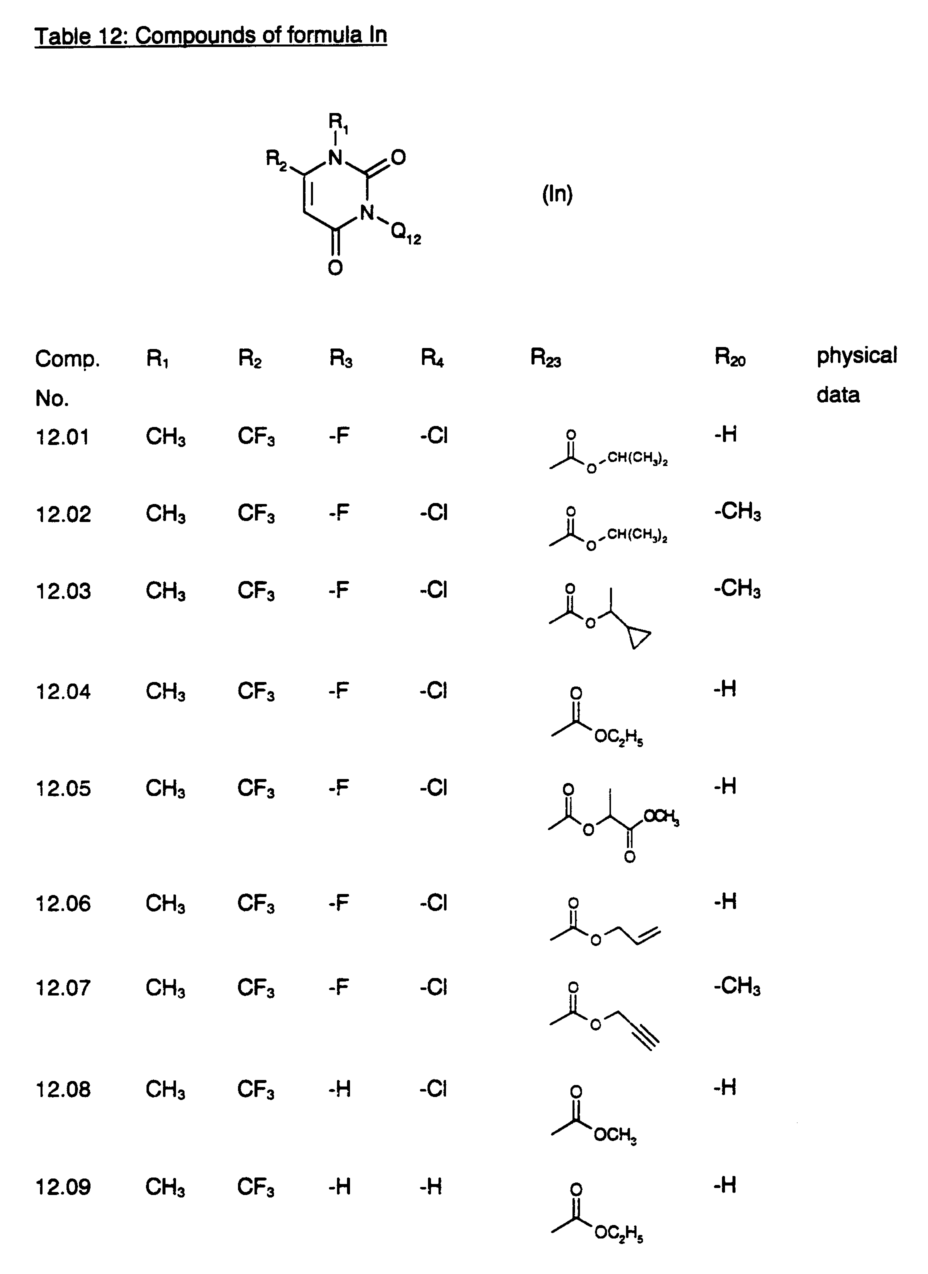

## Claims

1. Process for the production of compounds of formula I 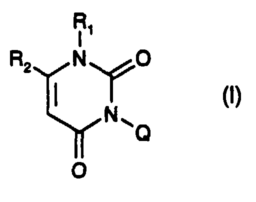 wherein
R₁ signifies methyl or ethyl;
R₂ signifies -CF₃, -CClF₂, -CCl₂F or -C₂F₅;
Q is a group 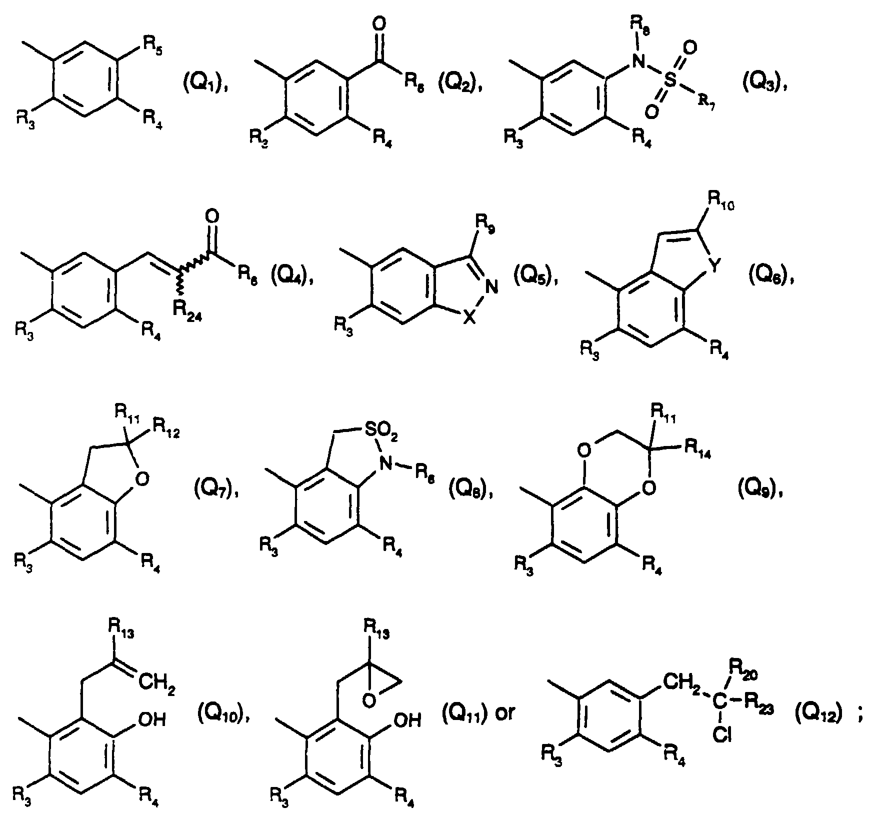
X and Y, independently of one another, are oxygen or sulphur;
R₃ signifies hydrogen, fluorine or chlorine;
R₄ signifies hydrogen, fluorine, chlorine, bromine, cyano, nitro, methyl or difluoromethyl;
R₅ signifies hydrogen, halogen, cyano, nitro, hydroxy, C₁₋₆-alkoxy, C₁₋₆-alkylthio, C₂₋₆-alkenyloxy, C₂₋₆-alkinyloxy, C₁₋₆-halogenalkoxy, C₂₋₆halogen-alkenyloxy, C₂₋₈-alkylcarbonylalkoxy, C₂₋₈-alkoxycarbonylalkoxy, C₁₋₃-alkyl-oxiranylmethoxy, 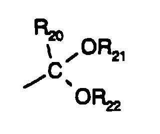 C₄₋₈-alkenyloxycarbonylalkoxy or C₄₋₈-alkinyloxycarbonylalkoxy;
R₂₀ is hydrogen or C₁₋₄-alkyl;
R₂₁ and R₂₂, independently of one another, signify C₁₋₄-alkyl; or R₂₁ and R₂₂ together signify a C₂₋₃-alkylene bridge;
R₂₃ signifies cyano or COR₆;
R₂₄ signifies hydrogen or halogen;
R₆ signifies OH, C₁₋₆-alkoxy, C₂₋₆-alkenyloxy, C₂₋₆-alkinyloxy, C₂₋₈-alkoxyalkoxy, C₃₋₆-cycloalkoxy, C₃₋₆-cycloalkenyloxy, C₃₋₆-cycloalkyl-C₁₋₆-alkoxy, C₁₋₆-halogen-alkoxy, C₂₋₆-halogen-alkenyloxy, C₁₋₆-hydroxycarbonylalkoxy, C₃₋₈-alkoxycarbonylalkoxy, C₃₋₈-alkenyloxycarbonylalkoxy, C₃₋₈-alkinyloxycarbonylalkoxy, N(C₁₋₃-alkyl)₂ or N(C₃₋₄-alkenyl)₂;
R₇ signifies C₁₋₆-alkyl, C₂₋₆-alkenyl, C₂₋₆-alkinyl, C₂₋₈-alkoxyalkyl, C₃₋₆-cycloalkyl, C₁₋₆-halogen-alkyl, C₁₋₆-halogen-alkenyl, C₂₋₆-alkylsulfonyloxyalkyl, C₁₋₁₀-phenylsulfonyloxyalkyl, N(C₁₋₅-alkyl)₂ or diallylamino;
R₈ signifies hydrogen, C₁₋₆-alkyl, C₂₋₆-alkenyl, C₂₋₆-alkinyl, C₂-C₁₀-alkoxyalkyl, C₃₋₆-cycloalkyl, C₃₋₆-cycloalkenyl, C₁₋₆-halogen-alkyl, C₃₋₆-halogen-alkenyl, C₂₋₆-hydroxycarbonylalkyl, C₃₋₈-alkoxycarbonylalkyl, C₃₋₈-alkenyloxycarbonylalkyl or C₃₋₈-alkinyloxycarbonylalkyl;
R₉ signifies hydrogen, OH, CH₂COOR₁₅, CH₂CON(C₁₋₄-alkyl)₂, CH₂CON(C₃₋₄-alkenyl)₂, COOR₁₆, CON(C₁₋₄-alkyl)₂, CON(C₃₋₄-alkenyl)₂, C₁₋₆-alkyl, hydroxy-C₁₋₆-alkyl, C₂₋₁₀-alkoxycarbonyl-alkoxy or C₂₋₈-alkoxyalkyl;
R₁₀ signifies hydrogen, Cyano, C₁₋₆-alkyl, C₂₋₆-alkenyl, C₂₋₆-alkinyl, hydroxy-C₁₋₆-alkyl, C₂₋₈-alkoxyalkyl, C₃₋₆-cycloalkyl, C₁₋₆-halogen-alkyl, COOR₁₇, CON(C₁₋₄-alkyl)₂ or CON(C₃₋₄-alkenyl)₂;
R₁₁ signifies hydrogen, C₁₋₆-alkyl or C₁₋₆-halogen-alkyl;
R₁₂ signifies hydrogen, C₁₋₆-alkyl, C₁₋₆-halogen-alkyl, CH₂OH, C₂₋₆-alkoxyalkyl, C₂₋₆-halogenalkoxyalkyl, COOR₁₈, CON(C₁₋₄-alkyl)₂ or CON(C₃₋₄-alkenyl)₂;
R₁₃ signifies hydrogen, C₃₋₁₆-trialkylsilyloxy, C₁₋₆-alkoxy, chlorine, C₁₋₆-alkyl, C₂₋₆-alkenyl, C₂₋₆-alkinyl, C₃₋₆-cycloalkyl, C₃₋₆-cycloalkenyl, C₁₋₆-halogen-alkyl, C₂₋₆-halogen-alkenyl or C₁₋₆-hydroxycarbonylalkyl;
R₁₄ signifies hydrogen, C₁₋₆-alkyl or C₁₋₆-halogenalkyl;
R₁₅ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₃₋₈-alkoxycarbonylalkyl, C₃₋₈-alkenyloxycarbonylalkyl or C₃₋₈-alkinyloxycarbonylalkyl;
R₁₆ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₃₋₈-alkoxycarbonylalkyl, C₃₋₈-alkenyloxycarbonylalkyl or C₃₋₈-alkinyloxycarbonylalkyl;
R₁₇ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₂₋₈-alkoxycarbonylalkyl, C₃₋₈-alkenyloxycarbonylalkyl or C₃₋₈-alkinyloxycarbonylalkyl; and
R₁₈ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₃₋₈-alkoxycarbonylalkyl, C₃₋₈-alkenyloxycarbonylalkyl or C₃₋₈-alkinyloxycarbonylalkyl, in such manner whereby a compound of formula II
O=C=N-Q (II)
wherein Q has the significance given under formula I, is reacted at a temperature of -5ºC to +40ºC with an enamine of formula III 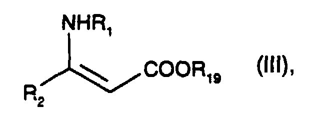 wherein R₁ and R₂ have the significances given under formula I,and R₁₉ signifies C₁-C₆-alkyl, in pure dimethylformamide, dimethyl sulphoxide, acetonitrile, propionitrile, ethyl acetate, tetrahydrofuran, dioxane, N-methylpyrrolidone, methyl-tert.-butylether, dimethylacetamide or toluene or mixtures thereof as solvents, in the presence of 0.2 to 0.4 equivalents of a base selected from potassium tert.butylate, sodium tert.butylate, sodium methylate, sodium ethylate, potassium methylate, potassium ethylate, sodium hydride, potassium hydride, sodium pentylate, potassium pentylate and 1,8-diazabicyclo[5.4.0]undec-7-ene (DBU).

2. Process according to claim 1 for the production of compounds of formula I, wherein R₁ signifies methyl or ethyl; R₂ signifies -CF₃, -CClF₂, -CCl₂F or -C₂F₅; Q is a group 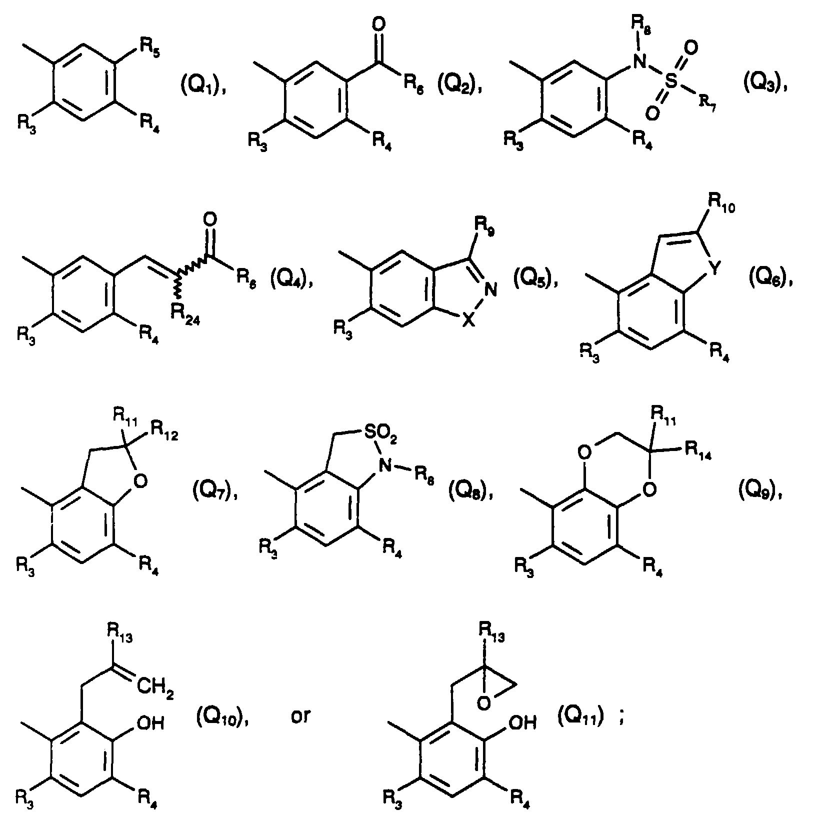
X and Y, independently of one another, are oxygen or sulphur;
R₃ signifies hydrogen, fluorine or chlorine;
R₄ signifies hydrogen, fluorine, chlorine, bromine, cyano, nitro, methyl or difluoromethyl;
R₅ signifies hydrogen, halogen, cyano, nitro, hydroxy, C₁₋₆-alkoxy, C₂₋₆-alkenyloxy, C₂₋₆-alkinyloxy, C₁₋₆-halogenalkoxy, C₂₋₆halogen-alkenyloxy, C₂₋₈-alkylcarbonyl-alkoxy or C₁₋₆-alkylthio;
R₆ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₁₋₆-hydroxycarbonylalkyl, C₃₋₈-alkoxycarbonylalkyl, C₃₋₈-alkenyloxycarbonylalkyl, C₃₋₈-alkinylcarbonylalkyl, C₂₋₆-dialkylenamino or C₃₋₈-dialkylenaminooxyalkyl;
R₇ signifies C₁₋₆-alkyl, C₂₋₆-alkenyl, C₂₋₆-alkinyl, C₂₋₈-alkoxyalkyl, C₃₋₆-cycloalkyl, C₁₋₆-halogen-alkyl, -C₁₋₆-halogen-alkenyl, C₂₋₆-alkylsulfonyloxyalkyl, C₁₋₁₀-phenylsulfonyloxyalkyl, C₂₋₁₀-dialkylamino or diallylamino;
R₈ signifies hydrogen, C₁₋₆-alkyl, C₂₋₆-alkenyl, C₂₋₆-alkinyl, C₃₋₆-cycloalkyl, C₃₋₆-cycloalkenyl, C₁₋₆-halogen-alkyl, -C₁₋₆-halogen-alkenyl, C₂₋₆-hydroxycarbonylalkyl, C₃₋₈-alkoxycarbonylalkyl, C₂₋₈-alkenyloxycarbonylalkyl or C₃₋₈-alkinylcarbonylalkyl;
R₉ signifies hydrogen, CH₂COOR₁₅, COOR₁₆, C₁₋₆-alkyl or C₂₋₈-alkoxyalkyl;
R₁₀ signifies hydrogen, C₁₋₆-alkyl, C₂₋₆-alkenyl, C₂₋₆-alkinyl, C₂₋₈-alkoxyalkyl, C₃₋₆-cycloalkyl, C₁₋₆-halogen-alkyl or COOR₁₇;
R₁₁ signifies hydrogen, C₁₋₆-alkyl or C₁₋₆-halogen-alkyl;
R₁₂ signifies hydrogen, C₁₋₆-alkyl, C₁₋₆-halogen-alkyl, CH₂OH, C₂₋₆-alkoxyalkyl, C₂₋₆-halogen-alkoxyalkyl or COOR₁₈;
R₁₃ signifies hydrogen, hydroxy, C₃₋₁₆-trialkylsilyloxy, C₁₋₆-alkoxy, chlorine, C₁₋₆-alkyl, C₂₋₆-alkenyl, C₂₋₆-alkinyl, C₃₋₆-cycloalkyl, C₃₋₆-cycloalkenyl, C₁₋₆-halogen-alkyl, C₂₋₆-halo-alkenyl or C₁₋₆-hydroxycarbonylalkyl;
R₁₄ signifies hydrogen, C₁₋₆-alkyl or C₁₋₆-halogen-alkyl;
R₁₅ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₃₋₈-alkoxycarbonylalkyl, C₃₋₈-alkenyloxycarbonylalkyl, C₃₋₈-alkinylcarbonylalkyl, C₂₋₆-dialkylamino, C₂₋₈-dialkyleneamino or C₃₋₈-dialkyleneaminooxyalkyl;
R₁₆ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₃₋₈-alkoxycarbonylalkyl, C₃₋₈-alkenyloxycarbonylalkyl, C₃₋₈-alkinylcarbonylalkyl, C₂₋₆-dialkylamino, C₂₋₈-dialkyleneamino or C₃₋₈-dialkyleneaminooxyalkyl;
R₁₇ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₃₋₈-alkoxycarbonylalkyl, C₃₋₈-alkenyloxycarbonylalkyl, C₃₋₈-alkinylcarbonylalkyl, C₂₋₆-dialkylamino, C₂₋₈-dialkyleneamino or C₃₋₈-dialkyleneaminooxyalkyl and
R₁₈ signifies hydrogen, C₁₋₆-alkyl, C₂₋₆-alkenyl, C₂₋₆-alkinyl, C₂₋₈-alkoxyalkyl, C₃₋₆-cycloalkyl, C₃₋₆-cycloalkenyl, C₁₋₆-halogen-alkyl, C₂₋₆-halogen-alkenyl, C₂₋₆-hydroxycarbonylalkyl, C₃₋₈-alkoxycarbonylalkyl, C₃₋₈-alkenyloxycarbonylalkyl, C₃₋₈-alkinylcarbonylalkyl, C₂₋₆-dialkylamino, C₂₋₈-dialkyleneamino or C₃₋₈-dialkyleneaminooxyalkyl, characterised in that a compound of formula II
O=C=N-Q (II)
wherein Q has the significance given under formula I, is reacted at a temperature of -5ºC to +40ºC with an enamine of formula III 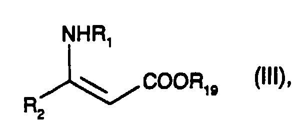 wherein R₁ and R₂ have the significances given under formula I,and R₁₉ signifies C₁-C₆-alkyl, in pure dimethylformamide or dimethyl sulphoxide as the solvent, in the presence of 0.2 to 0.4 equivalents of a base selected from potassium tert.butylate, sodium methylate and sodium hydride.

3. Process according to claim 1, characterised in that potassium tert.butylate is used.

4. Process according to claim 1, characterised in that the base is used in a quantity of 0.25 to 0.35 equivalents with respect to the employed enamine of formula III.

5. Process according to claim 4, characterised in that the base is employed in a quantity of 0.3 equivalents.

6. Process according to claim 1, characterised in that the reaction is carried out at a temperature of 0°C to +20°C.

7. Process according to claim 6, characterised in that the reaction is carried out at a temperature of 0°C to +5°C.

8. Process according to claim 1, characterised in that the reaction is carried out in a manner whereby the base is presented in pure dimethylformamide, dimethyl sulphoxide, acetonitrile, propionitrile, ethyl acetate, tetrahydrofuran, dioxane, N-methylpyrrolidone, methyl-tert.-butyl ether, dimethylacetamide or toluene, or mixtures thereof, and then the compound of formula III is added, and subsequently the compound of formula II is added.

9. Process according to claim 1 for the production of compounds of formula I, wherein Q denotes Q₁, Q₂, Q₃ or Q₄.

10. Process according to claim 1 for the production of
3-(2,5-difluoro-phenyl)-1-methyl-6-trifluoromethyl-1H-pyrimidine-2,4-dione,
3-(2,4-difluoro-phenyl)-1-methyl-6-trifluoromethyl-1H-pyrimidine-2,4-dione,
3-(4-chloro-2-fluoro-phenyl)-1-methyl-6-trifluoromethyl-1H-pyrimidine-2,4-dione,
3-(5-bromo-4-chloro-2-fluoro-phenyl)-1-methyl-6-trifluoromethyl-1H-pyrimidine-2,4-dione,
3-(4-chloro-2-fluoro-5-nitro-phenyl)-1-methyl-6-trifluoromethyl-1H-pyrimidine-2,4-dione,
3-(4-chloro-5-cyano-2-fluoro-phenyl)-1-methyl-6-trifluoromethyl-1H-pyrimidine-2,4-dione,
3-(5-methallyloxy-4-chloro-2-fluoro-phenyl)-1-methyl-6-trifluoromethyl-1H-pyrimidine-2,4-dione,
3-[4-chloro-2-fluoro-5-(2-methyl-oxiranylmethoxy)-phenyl]-1-methyl-6-trifluoromethyl-1H-pyrimidine-2,4-dione,
2-chloro-5-(3-methyl-2,6-dioxo-4-trifluoromethyl-3,6-dihydro-2H-pyrimidin-1-yl)-benzoic acid isopropyl ester,
2-chloro-4-fluoro-5-(3-methyl-2,6-dioxo-4-trifluoromethyl-3,6-dihydro-2H-pyrimidin-1-yl)-benzoic acid isopropyl ester,
2-chloro-5-(3-methyl-2,6-dioxo-4-trifluoromethyl-3,6-dihydro-2H-pyrimidin-1-yl)-benzoic acid 1-cyclopropyl-ethyl ester,
2-chloro-5-(3-methyl-2,6-dioxo-4-trifluoromethyl-3,6-dihydro-2H-pyrimidin-1-yl)-benzoic acid 1-methoxycarbonyl-ethyl ester,
2-chloro-5-(3-methyl-2,6-dioxo-4-trifluoromethyl-3,6-dihydro-2H-pyrimidin-1-yl)-benzoic acid 2-ethoxycarbonyl-1-methyl-ethyl ester,
2-chloro-5-(3-methyl-2,6-dioxo-4-trifluoromethyl-3,6-dihydro-2H-pyrimidin-1-yl)-benzoic acid 1-carboxy-1-methyl-ethyl ester,
2-chloro-5-(3-methyl-2,6-dioxo-4-trifluoromethyl-3,6-dihydro-2H-pyrimidin-1-yl)-benzoic acid 1-methyl-1-(1-methyl-allyloxycarbonyl)-ethyl ester,
2-chloro-5-(3-methyl-2,6-dioxo-4-trifluoromethyl-3,6-dihydro-2H-pyrimidin-1-yl)-benzoic acid 1-methoxycarbonyl-1-methyl-ethyl ester,
N-[2-chloro-4-fluoro-5-(3-methyl-2,6-dioxo-4-trifluoromethyl-3,6-dihydro-2H-pyrimidin-1-yl)-phenyl]-N-methyl-methanesulfonamide,
N-allyl-N-[2-chloro-4-fluoro-5-(3-methyl-2,6-dioxo-4-trifluoromethyl-3,6-dihydro-2H-pyrimidin-1-yl)-phenyl]-methanesulfonamide,
Ethanesulfonic acid [2-chloro-4-fluoro-5-(3-methyl-2,6-dioxo-4-trifluoromethyl-3,6-dihydro-2H-pyrimidin-1-yl)-phenyl]-ethyl-amide,
C-chloro-N-[2-chloro-4-fluoro-5-(3-methyl-2,6-dioxo-4-trifluoromethyl-3,6-dihydro-2H-pyrimidin-1-yl)-phenyl]-N-prop-2-ynyl-methanesulfonamide,
3-[2-chloro-4-fluoro-5-(3-methyl-2,6-dioxo-4-trifluoromethyl-3,6-dihydro-2H-pyrimidin-1-yl)-phenyl]-acrylic acid isopropyl ester,
3-[2-chloro-4-fluoro-5-(3-methyl-2,6-dioxo-4-trifluoromethyl-3,6-dihydro-2H-pyrimidin-1-yl)-phenyl]-acrylic acid ethyl ester,
3-[2-chloro-4-fluoro-5-(3-methyl-2,6-dioxo-4-trifluoromethyl-3,6-dihydro-2H-pyrimidin-1-yl)-phenyl]-acrylic acid allyl ester and
2-chloro-5-(3,6-dihydro-2,6-dioxo-3-methyl-4-trifluoromethyl-1-(2H)pyrimidinyl)-benzoic acid 1-allyloxycarbonyl-1-methyl-ethylester.
